# EUROPÄISCHE PATENTANMELDUNG

(11) **EP 0 875 239 A2**
(43) Veröffentlichungstag der Anmeldung: **04.11.1998**
(21) Anmeldenummer: 98107103.8
(22) Anmeldetag: 18.04.1998
(51) Int. Cl.: A61K 7/32, A61K 7/48

(54) **Verwendung von Estern aus Fettsäuren und Di- und Oligosacchariden gegen die Adhäsion von Mikroorganismen**

(30) Priorität: 03.05.1997 DE 19718777
(71) Anmelder: Beiersdorf Aktiengesellschaft, 20245 Hamburg (DE)
(72) Erfinder: Wolf, Florian, Dr., 20251 Hamburg (DE); Maurer, Peter, 22880 Wedel (DE); Schreiber, Jörg, Dr., 22087 Hamburg (DE); Bünger, Joachim, Dr., 22459 Hamburg (DE)

(57) **Zusammenfassung**

Verwendung einer Verbindung oder mehrerer Verbindungen aus der Gruppe der mit einem oder mehreren Fettsäureresten veresterten Di- und Oligosaccharide als antiadhäsive Wirkstoffe gegenüber Mikroorganismen, Viren, Parasiten und Protozoen.

## Beschreibung

Die Erfindung betrifft die Verwendung von Estern aus Fettsäuren und Disacchariden und Oligosacchariden gegen die Adhäsion von Mikroorganismen, einschließlich Viren und Pilzen sowie ferner Parasiten.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff 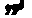 Antibiotika 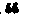 beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκηζ = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehitau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten - unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Protozoen sind parasitisch lebende Einzeller mit klar abgegreztem Zellkern, die sich ungeschlechtlich fortpflanzen (durch Zwei- oder Vierfachteilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozoiten) unter ungünstigen Umständen auch Zysten als Dauerformen ausbilden,

Je nach Fortbewegungsart und -apparat werden Protozoen in vier verschiedene Gruppen unterteilt:
(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweise durch Leishmania donovanii, L. tropica, L. brasiliensis, L. mexicana, L. diffusa oder L. pifanoi), Trypanosmiasis (verursacht durch verschiedene Trypanosoma-Arten), Amöbenruhr und Amöbiasis (verursacht beisielsweise durch verschiedene Entamoeba-Arten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erhablich, das menschliche Wohlbefinden. Es besteht daher bei den betroffenen Personen ein erheblicher bedarf, diesem Zustande abzuhelfen. Eine Aufgabe der vorliegenden Erfindung war es also, gegen Protozoen wirksame Wirkprinzipien zu finden.

Parasiten [παρασ 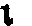 τοζ = grch. Tischgenosse, insb. Schmarotzer] sind ein- oder mehrzellige Pflanzen oder Tiere, die sich auf (= Ektoparasiten) oder in (= Endoparasiten) anderen Lebewesen auf deren kosten ernähren, und zwar mit (= Pathogene Parasiten) oder ohne (Apathogene Parasiten) Verursachung von Krankheitserscheinungen. Die Lebensweise ist entweder auch aprophytisch oder aber rein parasitär, eventuell nur als periodischer, temporärer oder stationärer Parasit. Die Entwicklung von Parasiten ist an einen oder mehrere verschiedene Wirtsorgansimen gebunden, wobei der Mensch Zwischenwirt oder Endwirt sein kann.

Medizinisch und dermatologisch bedeutsame Parasiten sind beispielsweise die Helminthen, die sich wiederum in Trematodae, Cestodae und Nematodae untergliedern. Das menschliche Wohlbefinden beeinträchtigende Helminthosen sind beispielsweise Bilharziose, (verursacht durch Schistosoma-Arten), Bandwurmbefall vom Darm und anderen inneren Organen (verursacht durch beispielsweise Taenia-Arten und Echinococcus-Arten), Ascariasis (verursacht durch Ascaris lumbricoides), Enterobiasis (verursacht durch Enterobium vermicularis), Paragonimiasis (verursacht durch Paragonium-Arten), Filariose (verursacht beispielsweise durch Wucheria bancrofti) sowie anderer Nematodenbefall (beispielsweise verursacht durch Trichuris trichura oder Trichinella spiralis).

Darüber hinaus bestehen eine Vielzahl auf bzw. in Mensch und Tier parasitisch lebender Insektenarten bzw. Spinnentieren, die medizinische und dermatologische Veränderungen der Wirtsorganismen hervorrufen. In dieser Hinsicht für die Beeinträchtigung des menschlichen Wohlbefindens verantwortliche Parasitosen sind beispielsweise Accrodermatitis (verursacht durch Getreidemilben, beispielsweise Pediculoides ventricosus), Skabies (verursacht durch Sarcoptes scabii), Fliegen- und/oder Fliegenlarvenbefall (verursacht beispielsweise durch Glossina-, Stomoxys-, Tabanus-, Chrysops-, Lucilia-, Chrysomya-, Cochliamya-, Wohlfartia-, Cordylobia- oder Dermatobia-Arten), Mücken- und/oder Mückenlarvenbefall (verursacht beispielsweise durch Aedes- Culex-, Anopheles-, Phlebotomus- Culicuides-, Sumilium- oder Haemagoges-Arten), Zeckenbefall (verursacht beispielsweise durch Argas persicus und andere Argas-Arten, Ornithodorus erraticus und andere Ornithodorus-Arten, Orobius- Rhiphocephalus-, Dermacentor-, Haemaphysalis-, Amblyomma-, Ixodes-Arten), Porocephalose (verursacht durch Porocephalus-Arten), Flohbefall (verursacht durch beispielsweise Pulex irritans, Ctenocephalides canis, Xenopsylla cheopsis, Nosophyllus fasciatus oder Sarcopsylla penetrans), Läusebefall (verursacht beispielsweise durch Phthirius pubis, Pediculosus humanus oder Pediculosus captits), Wanzenbefell (verursacht beispielsweise durch Cimex lectularius, Cimex hemipterus, Panstrongylus megistus, Rhodnius prolixus, Triatoma dimidata, Triatoma infestans, Triatoma sordida oder Triatoma brasiliensis) sowie Milbenbefall (verursacht beispielsweise durch Demodex folliculorum und andere Demodex-Arten sowie durch Dermamyskus-Arten, Glyciphagus domesticus, Pyemotes-Arten, Sarcoptes-Arten oder Trombicula-Arten).

Dabei ist von zusätzlicher Bedeutung, daß die auf oder im menschlichen Organismus lebenden Parsiten ihrerseits wieder Überträger von Bakterien, Mycota, Protozoen und Viren sein können, die Gesundheit und Wohlbefinden des Wirtsorganismus, beispielsweise des Menschen, nachhaltig beeintrachtigen können. Es bestand daher der Bedarf, gegen Parasitosen wirksame Wirkprinzipien zu finden, welche das medizinische oder dermatologische Erscheinungsbild zu verbessern imstande sind. Diesen Bedarf zu stillen, war daher eine weitere Aufgabe der vorliegenden Erfindung.

Im Gegensatze zu den prokaryotischen und eukaryotischen zellulären Organismen sind Viren [virus = lat. Gift] biologische Strukturen, welche zur Biosynthese eine Wirtszelle benötigen. Extrazelluläre Viren (auch 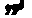 Virionen 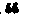 genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen ist jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden.

Dem Stande der Technik mangelt es jedenfalls an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, nämlich Substanzen zu finden, welche wirksam einen ein- oder mehrzelligen Organismus vor schädlichen Folgen einer Virusinfektion, sei es prophylaktisch oder therapeutisch, zu schützen.

Es ist bereits bekannt, Mikroorganismen, einschließlich Viren und Pilze sowie ferner Parasiten auf Oberflächen durch Mikrobizide abzutöten oder mit Reinigungsmitteln abzuspülen, um auf diese Weise ihre Anzahl auf der Fläche zu vermindern. Solche Oberflächen sind z.B. Organaußenflächen oder Organ-Oberflächen, insbesondere von der Haut oder Schleimhaut und Körperhöhlen oder Organhohlräumen, Wundhöhlen oder das Auge, bzw. die Augenhöhle, die Augenhornhaut und der Bereich zwischen Augapfel und Augenlid.

Beide Methoden haben bekannte Nachteile. So können z.B. Desinfektionsmittel die Oberflächen schädigen und Reinigungsmittel reichen oft in der Wirkung nicht aus.

Aufgabe der Erfindung war es daher, eine schonende und wirkungsvolle Methode zu schaffen, mit der es gelingt, die Anzahl von Mikroorganismen, Viren, Parasiten und Protozoen auf Oberflächen gering zu halten oder zu verringern oder zu verhindern, daß Mikroorganismen, Parasiten und Protozoen an Oberflächen haften.

Mit den Begriffen 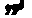 antiadhäsiv 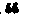 und 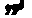 antiadhäsiver Wirkung 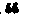 der erfindungsgemäßen Wirkstoffe ist gemeint, daß die Adhäsion von Mikroorganismen, Viren, Parasiten und Protozoen an Oberflächen, und zwar an belebten Oberflächen (z.B. Körpergewebe) wie auch an unbelebten Oberflächen (z.B. Gegenstände des täglichen Bedarfes) herabgesetzt oder aufgehoben ist.

Erfindungsgemäß werden all diese Aufgaben gelöst durch die Verwendung einer Verbindung oder mehrerer Verbindungen aus der Gruppe der mit einem oder mehreren Fettsäureresten veresterten Di- und Oligosaccharide als antiadhäsive Wirkstoffe gegenüber Mikroorganismen, Viren, Parasiten und Protozoen. Die erfindungsgemäß verwendeten Wirkstoffe sind zur Behandlung sowie zur Prophylaxe der durch die die genannten Mikroorganismen, Viren, Parasiten und Protozoen hervorgerufenen Störungen und Krankheiten geeignet.

Zwar werden in der deutschen Offenlegungsschrift 195 03 423 antiadhäsive Wirkstoffe auf der Grundlage von Zuckern beschrieben. Die erfindungsgemäße Verwendung von Di- und Oligosaccharidestern von Fettsäuren, welche sich durch überraschende Überlegenheit gegenüber dem Stand der Technik auszeichnen, konnten durch die vorab erwähnt Schrift nicht nahegelegt werden.

Gegenstand der Erfindung ist auch die Verwendung einer Verbindung oder mehrerer Verbindungen aus der Gruppe der mit einem oder mehreren Fettsäureresten veresterten Di- und Oligosaccharide als antiadhäsive Wirkstoffe gegenüber Mikroorganismen, Viren, Parasiten und Protozoen als Bestandteil von kosmetischen oder dermatologischen Zubereitungen sowie von Zubereitungen, die zur Dekontamination von belebten und unbelebten Oberflächen dienen.

Die erfindungsgemäß verwendeten antiadhäsiven Wirkstoffe und Zubereitungen, diese Wirkstoffe enthaltend, können prophylaktisch verwendet werden und bewirken, daß sich auf belebten und unbelebten Oberflächen nur noch geringe und keine störenden Ansammlungen von Mikroorganismen, Parasiten und Protozoen ausbilden, oder sie verdrängen auch bereits anhaftende Mikroorganismen, Parasiten und Protozoen von der Oberfläche und verringern so deren Anzahl.

Als Kohlehydrat-Komponenten der erfindungsgemäß verwendeten Ester aus Fettsäuren und Disacchariden und Oligosacchariden, die sprachlich kurzgefaßt auch gelegentlich unter die Bezeichnung 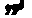 Kohlenhydrat-Ester 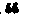 fallen sollen, sind alle Zuckerreste enthaltende linearen und verzweigt-kettigen Verbindungen geeignet, welche aus mindestens zwei Monosaccharidbestandteilen zusammengesetzt sind. Als niedrigste 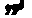 Oligosaccharide 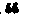 im Sinne der vorliegenden Erfindung werden Trisaccharide, also Zuckerreste enthaltende Verbindungen geeignet, welche aus drei Monosaccharid-bestandteilen zusammengesetzt sind . Zu den Zuckern zählen insbesondere auch jeweils die Desoxy- und Didesoxy-Formen.

Als Kohlenhydrat-Komponenten der beanspruchten ein- bis sechfachen Ester aus Fettsäuren und Kohlenhydraten werden die folgenden Zuckerstrukturen bevorzugt:

### 1. Disaccharide

Gut geeignete Disaccharide sind z.B. die durch binäre Verknüpfungen obiger Monosaccharide gebildeten Disaccharide. Die Verknüpfung kann als α- oder β-glycosidische Bindung zwischen den beiden Untereinheiten erfolgen. Saccharose, Maltose, Lactobiose und Cellobiose werden bevorzugt.

### 2. Oligosaccharide

Gut geeignete Oligosaccharide bestehen aus mehreren, z.B. 2 - 7 Zuckereinheiten, vorzugsweise der unter 1 beschriebenen Zucker, insbesondere aus 2 bis 4 Einheiten in den bekannten, durch Kondensation entstandenen Bindungsformen und wie vorstehend genannt. Besonders bevorzugte Oligosaccharide sind - neben den Disacchariden - die Tri- und Tetrasaccharide.

Als Fettsäurekomponente der beanspruchten ein- bis sechfachen Ester aus Fettsäuren und Kohlenhydraten können gesättigte und/oder ein- bis fünffach ungesättigte Mono-Di-, Tri-, Tetra-, Penta- und Hexacarbonsäuren verwendet werden. Die gesättigten oder ein- bis fünffach ungesättigten Alkan- Alken- Alkadien- Alkatrien-, Alkatetraen-, Alkapentaen-, Alkin-, Alkadiin-, Alkatriin-, Alkatetrain-, Alkapentain sowie gemischten En-In-Säuren können unverzweigte oder ein- oder mehrfach verzweigte Alkylketten der Kettenlängen C₁ bis C₄₀ besitzen, die ihrerseits durch Arylgruppen, Hydroxyl-, Keto-, Amino- und/oder Sulfhydrylgruppen derivatisiert sein können. Als Acyl-Komponenten werden unverzweigte und verzweigtkettige Alkancarbonsäuren der Kettenlängen C1 bis C₄₀, z.B. Ameisensäure, Essigsäure, Propionsäure, Buttersäure, Valeriansäure, Capronsäure, Heptansäure, Octansäure, Nonansäure, Decansäure, Undecansäure, Laurinsäure, Tridecansäure, Myristinsäure, Pentadecansäure, Palmitinsäure, Heptadecansäure und Stearinsäure, deren Mono- und Oligo-hydroxyderivate z.B. Milchsäure, alpha-Hydroxy-Palmitinsäure, Wollwachs-säure, sowie ein- und mehrfach ungesättigte Alkencarbonsäuren der Ketten-längen C₃ bis C₄₀, z.B. Hexadecensäure, Rizinolsäure, Ölsäure, Linolsäure, Linolensäure oder Arachidonsäure, bevorzugt.

Die Liste der Fettsäurekomponenten der erfindungsgemäß verwendbaren Kohlenhydrat-Ester soll selbstverständlich nicht limitierend sein.

Der Veresterungsgrad der beanspruchten Wirksysteme reicht von der einfachen bis zur vollständigen Veresterung homo- oder heterolog veresterter Kohlenhydrate in sämtlichen möglichen Stellungs- und Stereoisomeren sowie Enantiomeren.

Erfindungsgemäß bevorzugte Zuckerester sind Ester aus Fettsäuren und Saccharose (in dieser Schrift gelegentlich auch 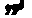 Sucrose 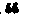 genannt), einfach bis vollständig verestert sowie kommerziell erhältliche kosmetische Rohstoffe, dieselben enthaltend.

Saccharose hat die nachfolgend gekennzeichnete Struktur:

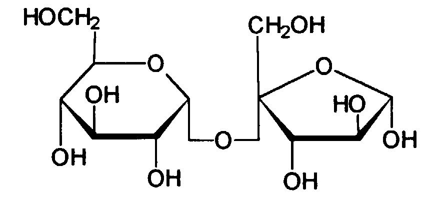

Ihre Ester, die erfindungsgemäß vorteilhaft Verwendung finden können, sind gekennzeichnet durch die Struktur 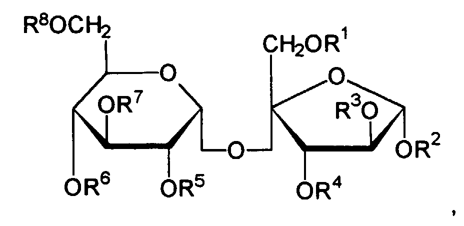 wobei R¹ - R⁸ vorteilhaft unabhängig voneinander gewählt werden können aus der Gruppe aus Wasserstoffatomen und verzweigten und unverzweigten Alkanoylgruppen mit 1 - 24 Kohlenstoffatomen. Insbesondere vorteilhaft ist es, wenn bis zu sieben der Reste R¹ - R⁸ Wasserstoffatome und einer bis zu acht der der Reste R¹ - R⁸ gewählt werden aus der Gruppe der verzweigten und unverzweigten Alkanoylgruppen mit 8 - 20 Kohlenstoffatomen, davon insbesondere der Lauroylrest und der Myristoylrest.

Zuckerester aus Saccharose und Fettsäuren sind beispielsweise erhältlich bei den Firmen Grillo Cosmetics [z.B. Grilloten LSE 65 K (Ester aus Saccharose und Kokosfettsäuren), Grilloten LSE 65 K soft (Ester aus Saccharose und Kokosfettsäuren), Grilloten LSE 87 K (Ester aus Saccharose und Kokosfettsäuren), Grilloten LSE 87 K *soft* (Ester aus Saccharose und Kokosfettsäuren) ode Grilloten PSE 141 G (Ester aus Saccharose und Palmitinsäure/ Stearinsäure)], Sisterna [z.B. Sisterna SP 70 C (Saccharosepalmitat/stearat), Sisterna SP 50 C (Saccharose-palmitat/stearat), Sisterna SP 30 C (Saccharosepalmitat/stearat), Sisterna SP 10 C (Saccharosepalmitat/stearat), Sisterna SP 01 C (Saccharosedistearat) oder Sisterna L 70 C (Saccharoselaurat)], und Mitsubishi Kagaku Foods Corporation [z.B. Ryoto Sugar Ester L 1695 (Sucroselaurat), Ryoto Sugar Ester M 1695 (Sucrosemyristat), Ryoto Sugar Ester M 595 (Gemisch aus Sucrose-mono-, di- und trimyristat), Ryoto Sugar Ester P 1570 (Sucrosepalmitat), Ryoto Sugar Ester S-570 (Sucrosedistearat), Ryoto Sugar Ester S-370 (Sucrose-tristearat), Ryoto Sugar Ester S-270 (Sucrosetetrastearat).

Die erfindungsgemäßen Wirkstoffe können einzeln eingesetzt werden. Es ist aber auch möglich, zwei, drei oder auch mehrere Wirkstoffe zusammen zu verwenden. Insbesondere können Ester aus Fettsäuren und Monosacchariden sowie Oligosaccharide kombiniert werden, wobei jeweils ein Saccharid aber auch zwei oder drei oder mehrere Zucker gewählt werden können. Zusammen mit den vorstehend genannten Zuckern oder deren Kombinationen können vorteilhaft ein Polysaccharid oder auch mehrere Polysaccharide verwendet werden.

Die genannten Zucker können insbesondere auch in ihrer Desoxy- oder Didesoxy-Form und insbesondere auch in der Form der erfindungsgemäßen Derivate vorliegen. Dies gilt auch für die folgenden bevorzugten Zuckerester: Grilloten LSE 65 K (Ester aus Saccharose und Kokosfettsäuren; Grillo Cosmetics), Grilloten LSE 65 K soft (Ester aus Saccharose und Kokosfettsäuren; Grillo Cosmetics), Grilloten LSE 87 K (Ester aus Saccharose und Kokosfettsäuren; Grillo Cosmetics), Grilloten LSE 87 K soft (Ester aus Saccharose und Kokosfettsäuren; Grillo Cosmetics), Grilloten PSE 141 G (Ester aus Saccharose und Palmitinsäure/Stearinsäure; Grillo Cosmetics), Sisterna SP 70 C (Saccharosepalmitat/stearat), Sisterna SP 50 C (Saccharose-palmitat/stearat), Sisterna SP 30 C (Saccharosepalmitat/stearat), Sisterna SP 10 C (Saccharosepalmitat/stearat), Sisterna SP 01 C (Saccharosedistearat), Sisterna L 70 C (Saccharoselaurat), Ryoto Sugar Ester L 1695 (Sucroselaurat; Mitsubishi Kagaku Foods Corporation), Ryoto Sugar Ester M 1695 (Sucrosemyristat; Mitsubishi Kagaku Foods Corporation), Ryoto Sugar Ester M 595 (Gemisch aus Sucrose-mono-, di- und trimyristat; Mitsubishi Kagaku Foods Corporation), Ryoto Sugar Ester P 1570 (Sucrosepalmitat; Mitsubishi Kagaku Foods Corporation), Ryoto Sugar Ester S-570 (Sucrosedistearat; Mitsubishi Kagaku Foods Corporation), Ryoto Sugar Ester S-370 (Sucrose-tristearat; Mitsubishi Kagaku Foods Corporation), Ryoto Sugar Ester S-270 (Sucrose-tetrastearat; Mitsubishi Kagaku Foods Corporation).

Besonders bevorzugt werden Kombinationen, insbesondere Kombinationen von mindestens zwei Wirkstoffen, die mindestens einen Fettsäureester eines Disaccharids oder mindestens einen Fettsäureester eines Trisaccarids enthalten, wobei diese auch jeweils in der Form der erfindungsgemäßen Derivate oder auch in der Desoxy- oder Didesoxy-Form vorliegen können.

Weiterhin werden Kombinationen, insbesondere Kombinationen von mindestens zwei Wirkstoffen bevorzugt, die Saccharose enthalten, wobei diese auch jeweils in der Form der erfindungsgemäßen Derivate vorliegen kann.

Besonders vorteilhaft werden Zwei- und Mehrstoffkombinationen gewählt, wobei die jeweiligen Einzelkomponenten vorteilhaft gewählt werden aus der Gruppe der folgenden Wirkstoffe: Grilloten LSE 65 K (Ester aus Saccharose und Kokosfettsäuren; Grillo Cosmetics), Grilloten LSE 65 K *soft* (Ester aus Saccharose und Kokosfettsäuren; Grillo Cosmetics), Grilloten LSE 87 K (Ester aus Saccharose und Kokosfettsäuren; Grillo Cosmetics), Grilloten LSE 87 K *soft* (Ester aus Saccharose und Kokosfettsäuren; Grillo Cosmetics), Grilloten PSE 141 G (Ester aus Saccharose und Palmitinsäure/ Stearinsäure; Grillo Cosmetics), Sisterna SP 70 C (Saccharosepalmitat/ stearat), Sisterna SP 50 C (Saccharosepalmitat/stearat), Sisterna SP 30 C (Saccharosepalmitat/stearat), Sisterna SP 10 C (Saccharosepalmitat/stearat), Sisterna SP 01 C (Saccharose-distearat), Sisterna L 70 C (Saccharoselaurat), Ryoto Sugar Ester L 1695 (Sucroselaurat; Mitsubishi Kagaku Foods Corporation), Ryoto Sugar Ester M 1695 (Sucrosemyristat; Mitsubishi Kagaku Foods Corporation), Ryoto Sugar Ester M 595 (Gemisch aus Sucrose-mono-, di- und trimyristat; Mitsubishi Kagaku Foods Corporation), Ryoto Sugar Ester P 1570 (Sucrosepalmitat; Mitsubishi Kagaku Foods Corporation), Ryoto Sugar Ester S-570 (Sucrose-distearat; Mitsubishi Kagaku Foods Corporation), Ryoto Sugar Ester S-370 (Sucrose-tristearat; Mitsubishi Kagaku Foods Corporation), Ryoto Sugar Ester S-270 (Sucrose-tetrastearat; Mitsubishi Kagaku Foods Corporation).

Bevorzugt werden auch die jeweiligen Einzelkomponenten der Kombinationen und die mit jeweils zwei Komponenten zu bildenden Zweier-Kombinationen aus den drei Wirkstoffen jeweils einer Dreier-Kombination.

Vorteilhaft können auch jeweils mit einem Fettsäureester eines Zuckers oder mehrerer Zuckern aus der Gruppe der Monosaccharide und/oder der Oligosaccharide ein oder mehrere Fettsäureester von Zuckern aus der Gruppe der Zuckerphosphate und/oder der Aminozucker und Acetylaminozucker kombiniert werden.

In den Kombinationen können die Wirkstoffe z.B. mit gleichen Gewichtsmengen oder auch z.B. im Gewichtsverhältnis von 1 : 100 bis 100 : 1, vorzugsweise 1 : 10 bis 10 : 1 verwendet werden, jeweils bezogen auf eine andere Komponente oder mehrere andere Komponenten.

Zubereitungen, insbesondere topische Zubereitungen, z.B. kosmetische und dermatologische Zubereitungen, mit den erfindungsgemäßen Wirkstoffen können diese z.B. in Mengen von 0,01 bis 99 Gew.-%, vorzugsweise 1 bis 50 Gew.-%, insbesondere aber 5 bis 20 Gew.-%, jeweils bezogen auf das Gesamtgewicht der Zubereitungen, enthalten. Insbesondere gelten diese Mengen auch jeweils für die Einzelkomponenten der Kombinationen.

Überraschenderweise wurde gefunden, daß die erfindungsgemäßen Wirkstoffe und Zubereitungen, die sie enthalten, die Adhäsion, d.h. das Vermögen der Mikroorganismen, Parasiten und Protozoen an Oberflächen anzuhaften, herabsetzen, so daß sich deren übliche Anzahl auf solche Flächen verringert, oder auch, daß sich keine oder keine wesentlichen Mengen von Mikroorganismen, Parasiten und Protozoen mehr nachweisen lassen.

Insbesondere die folgenden Mikroorganismen, Parasiten und Protozoen beispielsweise und die durch sie hervorgerufenen Störungen und Krankheiten können erfindungsgemäß besonders gut, insbesondere topisch behandelt werden:

### 1. Grampositive Bakterien

### Als Beispiele:

Fakultativ pathogene und pathogene Micrococaceae, insbesondere Staphylococcus epidermidis, z.B. bei der Entstehung von Achselgeruch und beim atopischen Ekzem sowie Staphylococcus aureus als wichtiges Pathogen, z.B. beim atopischen Ekzem, bei Neurodermitis und Psoriasis, Corynebacterium spec., z.B. bei der Entstehung des Achselgeruches, Propionibacterium spec., z.B. bei der Entstehung von Akne und bei unreiner Haut.

### 2. Gramnegative Bakterien

### Als Beispiele:

Escherichia coli, z.B. bei Colitis ulcerosa; Pseudomonas aeruginosa, z.B. bei Superinfektionen offener Wunden, z.B. im Bauchraum sowie bei cystischer Fibrosis, Enterococcaceae, z.B. bei Magen-Darm-Infektionen. Die Wirkstoffe können bei Spülungen und peroral verwendet werden.

### 3. Hefen

### Als Beispiele:

Pityrosporum ovale, z.B. verantwortlich für Schuppenbildung und z.B. bei Pityriasis versicolor, Pityrosporum-Follikulitis, seborrhoischem Ekzem, Psoriasis, kutanen und systemischen Mykosen, bei Aids und verwandten Erkrankungen, Candida albicans, z.B. verantwortlich für die Entstehung der kutanen Candidiasis. Die Wirkstoffe können topisch, parenteral, aber auch peroral verabreicht werden. Bevorzugt wird die intravasale Gabe, z.B. als Infusion oder Injektion.

### 4. Pilze

### Als Beispiele:

Mucor spec., z.B. verantwortlich für Mucor-Mycosen und Soor; Aspergillus niger, z.B. verantwortlich für kutane Aspergillose sowie Cryptococcus neoformans, z.B. bei Cryptococcoidose. Die Wirkstoffe können, wie bei 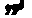 Hefen 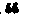 beschrieben, verabreicht werden.

### 5. Dermatophyten

### Als Beispiele:

Trichophyton rubrum, Trichophyton mentagrophytes, Epidermophyton spec., z.B. verantwortlich für die Entstehung von Dermatophytosen, z.B. Fußpilz. Die Wirkstoffe können, wie bei 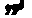 Hefen 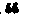 beschrieben, verabreicht werden.

### 6. Viren

### Als Beispiele:

Herpes Simplex Virus Typ 1 und 2, Varicella Zoster Virus (Gürtelrose); Epstein- Barr-Virus (Pfeiffer-Drüsenfieber), Cytomegalie-Virus, Warzen-Viren und Papilloma-Viren aber auch andere bekannte Viren. Die Wirkstoffe können wie bei 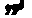 Hefen 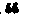 beschrieben verabreicht werden.

### 7. Parasiten und Protozoen

### Als Beispiele:

Trichomonas vaginalis, Lamblia intestinalis, Leishmania donovanii, L. tropica, L. brasiliensis, L. mexicana, L. diffusa oder L. pifanoi, verschiedene Trypanosoma-Arten, verschiedene Entarnoeba-Arten, Jodamoeba butschlii, Naegleria fowleri, Isospora belli, Balantidium coli, Schistosoma-Arten, Taenia-Arten und Echinococcus-Arten, Ascaris lumbricoides, Enterobium vermicularis, Paragonium-Arten, Wucheria bancrofti sowie Nematodenbefall, beispielsweise verursacht durch Trichuris trichura oder Trichinella spiralis, Getreidemilben, beispielsweise Pediculoides ventricosus, Sarcoptes scabii, Glossina-, Stomoxys-, Tabanus-, Chrysops-, Lucilia-, Chrysomya-, Cochliamya-, Wohlfartia-, Cordylobia- oder Dermatobia-Arten, Aedes- Culex-, Anopheles-, Phlebotomus- Culicuides-, Sumilium- oder Haemagoges-Arten, Argas persicus und andere Argas-Arten, Ornithodorus erraticus und andere Ornithodorus-Arten, Orobius- Rhiphocephalus-, Dermacentor-, Haemaphysalis-, Amblyomma- oder Ixodes-Arten, Porocephalus-Arten, Pulex irritans, Ctenocephalides canis, Xenopsylla cheopsis, Nosophyllus fasciatus oder Sarcopsylla penetrans, Phthirius pubis, Pediculosus humanus oder Pediculosus captits, Cimex lectularius, Cimex hemipterus, Panstrongylus megistus, Rhodnius prolixus, Triatoma dimidata, Triatoma infestans, Triatoma sordida oder Triatoma brasiliensis, Demodex folliculorum und andere Demodex-Arten sowie Dermamyskus-Arten, Glyciphagus domesticus, Pyemotes-Arten, Sarcoptes-Arten oder Trombicula-Arten. Die Wirkstoffe können wie bei "Hefen" beschrieben verabreicht werden.

Die Anwendung der Wirkstoffe kann topisch, perkutan, transdermal, parenteral, oral oder auch intravasal erfolgen.

Zubereitungen, die erfindungsgemäße Wirkstoffe enthalten, können topische Zubereitungen sein, beispielsweise kosmetische und dermatologische topische Zubereitungen oder aber auch übliche Arzneimittel-Darreichungsformen. Bevorzugt werden Desodorantien oder desodorierende Körperreinigungsprodukte oder Körperpflegeprodukte. Die Wirkstoffe können aber auch in Desinfektionsmitteln und/oder Reinigungsmitteln enthalten sein, die nicht nur zur Behandlung des Körpers oder der Haut bestimmt sind, sondern auch zum Reinigen und Desinfizieren von harten Oberflächen, medizinischen Materialien, Geräten, Instrumenten, Mobiliar und Wänden.

Für den Körper bestimmte Reinigungsmittel, Desinfektionsmittel, Spülmittel können ebenfalls zur Behandlung von der Haut verwendet werden wie schon die topischen Zubereitungen. Sie dienen aber vorzugsweise zur Behandlung von Körperhöhlen, Wunden und auch des Mund- und Rachenraumes sowie der Nase.

Die Wirkstoffe gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten, oder auch als Zäpfchen, Vaginalkugeln oder parenteral z.B. in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Prophylenglycol, Ether des Tetrahydrofurfurylalkohols und Wasser.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitver-wendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1 - 90 Gew.-% enthalten. Kapseln werden besonders bevorzugt. Einzeldosen enthalten die Wirkstoffe vorzugsweise in einer Menge von 0,1 bis 10 g.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Sofern die kosmetische oder dermatologische Zubereitung eine Lösung oder Lotion darstellt, können als Lösungsmittel verwendet werden: Wasser oder wäßrige Lösungen, ferner Öle, wie Triglyceride der Caprin- oder der Caprylsäure, vorzugsweise aber Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, aber auch Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet, so wie bei alkoholischen Lösungsmitteln Wasser ein vorteilhafter weiterer Bestandteil sein kann.

Kosmetische und dermatologische Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Die erfindungsgemäßen Wirkstoffe können auch besonders vorteilhaft in Mikroemulsionen, beispielsweise wie in der deutschen Offenlegungsschrift DE-195 9 079 beschrieben, verwendet werden.

Kosmetische und dermatologische Zubereitungen gemäß der Erfindung können besonders vorteilhaft als
a) unverdickte,
b) klassisch, z.B. durch Zusatz von Polyoxameren, Pluronics, Carragenanen oder Pflanzengummen verdickte,
c) durch Zusatz von A-B-A-Triblockcopolymeren (z.B. PEG-150-Distearat, Gesellschaft Akzo Nobel) oder α,ω-bis-polyethoxylierte Silane oder Silikone) verdickte,
d) durch Zusatz von Sternpolymeren (z.B. PEG-300-Pentaerythrityl-tetrastearat oder hydrophob modifizierte Tetrakis-polyethoxylierte Silane und Silikone) verdickte,
e) durch Zusatz von A-B-A-B-Multiblock-Copolymeren, Starburst-Polymeren, Dendrimeren und anderen supramolekularen Vernetzern (z.B. Rheodol TWIS 399, Gesellschaft KAO, oder PEG-120-Methylglucose-dioleat) verdickte Öl-in-Wasser- (O/W-), bikontinuierliche oder Wasser-in-Öl- (W/O-) Mikroemulsionen Verwendung finden.

Falls die erfindungsgemäße Verwendung in verdickten Mikroemulsionen verwirklicht werden soll, so ist es besonders vorteilhaft, von der Lehre der Schrift WO96/28132 Gebrauch zu machen.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind auch solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Die Zusätze der UVA-Filter und UVB-Filter oder Pigmente sind auch zur Stabilisierung der Zubereitungen geeignet.

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können als Sonnenschutzmittel dienen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornyliden-methyl)- sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.-Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden. Die Liste der verwendbaren UVA-Filtersubstanzen, die erfindungsgemäß Verwendung finden können, soll selbstverständlich nicht limitierend sein.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden. Die Liste der verwendbaren anorganischen Pigmente, die erfindungsgemäß Verwendung finden können, soll selbstverständlich nicht limitierend sein.

Die erfindungsgemäßen kosmetischen Zubereitungen können ferner kosmetische Hilfsstoffe enthalten, wie sie üblicherweise in solchen Zubereitungen verwendet werden, z.B. Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Erfindungsgemäß können als günstige Antioxidantien alle für kosmetische und/oder dermatologische Anwendungen geeigneten oder gebräuchlichen Anti-oxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure); Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Deri-vate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwassertoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe können dienen: Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine erfindungsgemäße ethoxylierte oder propoxylierte organische Verbindung im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Kosmetischen und/oder dermatologischen Reinigungszubereitungen im Sinne der vorliegenden Erfindung können einfache und kostengünstige Rezepturen zugrundeliegen. Sie haben neben ihrer die Erfindung begründenden Wirkung gleichzeitig eine gute Schaumentwicklung und eine hohe Reinigungskraft. Sie enthalten vorteilhaft eines oder mehrere Tenside der folgenden vier Gruppen A bis D:

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10 Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside. Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Benzalkoniumchlorid, Alkylbetain, Alkylamidopropylbetain und Alkyl-amidopropylhydroxysultain.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Die Reinigungsemulsionen enthalten im Sinn der vorliegenden Erfindung besonders vorteilhaft eines oder mehrere Tenside aus den Gruppen der anionischen und/oder amphoteren Tenside.

Vorteilhaft ist ferner die Verwendung einer Kombination von anionischen und/oder amphoteren Tensiden mit einem oder mehreren nicht-ionischen Tensiden.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben.

Kosmetische und dermatologische Zubereitungen zur Behandlung und Pflege der Haare können als Emulsionen vorliegen, die vom nicht-ionischen oder anionischen Typ sind. Nichtionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nichtionischem Charakter.

Kosmetische und dermatologische Zubereitungen zur Behandlung und Pflege der Haut oder der Haare können als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglycol und Polyethylenglycolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Soweit nicht anders angegeben, beziehen sich Mengenangaben, Prozentangaben und Teile auf das Gewicht, insbesondere auf das Gesamtgewicht der jeweiligen Mischung oder Zubereitung.

Gegenstand der Erfindung ist auch die Verwendung der erfindungsgemäß verwendeten Wirkstoffe zur Herstellung von Zubereitungen, insbesondere pharmazeutischen Zubereitungen zur Behandlung der angegebenen Störungen oder Krankheiten.

Die folgenden Beispiele sollen die Verkörperungen der vorliegenden Erfindungen verdeutlichen.

| Beispiel 1 | | | |
|---|---|---|---|
| W/O-Crème | I | II | III |
| Paraffinöl | 10,00 | 10,00 | 10,00 |
| Ozokerit | 4,00 | 4,00 | 4,00 |
| Vaseline | 4,00 | 4,00 | 4,00 |
| pflanzliches Öl | 10,00 | 10,00 | 10,00 |
| Wollwachsalkohol | 2,00 | 2,00 | 2,00 |
| Aluminiumstearat | 0,40 | 0,40 | 0,40 |
| Grilloten LSE 65 K | 1,00 | - | - |
| Sisterna SP 70 C | - | 1,00 | - |
| Ryoto Sugar Ester M 595 | - | - | 1,00 |
| Parfum, Konservierungsstoffe | q.s. | q.s. | q.s. |
| Wasser, VES | ad 100,00 | ad 100,00 | ad 100,00 |

| Beispiel 2 | | | |
|---|---|---|---|
| W/O-Lotion | I | II | III |
| Paraffinöl | 25,00 | 25,00 | 25,00 |
| Siliconöl | 2,00 | 2,00 | 2,00 |
| Ceresin | 1,50 | 1,50 | 1,50 |
| Wollwachsalkohol | 0,50 | 0,50 | 0,50 |
| Glucosesesquiisostearat | 2,50 | 2,50 | 2,50 |
| Grilloten PSE 141 G | 1,00 | - | - |
| Sisterna SP 01 C | - | 1,00 | - |
| Octaacetylsaccharose | - | - | 1,00 |
| Parfum, Konservierungsstoffe | q.s. | q.s. | q.s. |
| Wasser, VES | ad 100,00 | ad 100,00 | ad 100,00 |

| Beispiel 3 | | | |
|---|---|---|---|
| O/W-Lotion | I | II | III |
| Paraffinöl | 5,00 | 5,00 | 5,00 |
| Isopropylpalmitat | 5,00 | 5,00 | 5,00 |
| Cetylalkohol | 2,00 | 2,00 | 2,00 |
| Bienenwachs | 2,00 | 2,00 | 2,00 |
| Ceteareth-20 | 2,00 | 2,00 | 2,00 |
| PEG-20-Glycerylstearat | 1,50 | 1,50 | 1,50 |
| Glycerin | 3,00 | 3,00 | 3,00 |
| Octaacetylsaccharose | 1,00 | - | - |
| Sisterna SP 10 C | - | 1,00 | - |
| Saccharoselaurat | - | - | 1,00 |
| Parfum, Konservierungsstoffe | q.s. | q.s. | q.s. |
| Wasser, VES | ad 100,00 | ad 100,00 | ad 100,00 |

| Beispiel 4 | | | |
|---|---|---|---|
| O/W-Crème | I | II | III |
| Pflanzliches Öl | 10,00 | 10,00 | 10,00 |
| Cetylalkohol | 2,00 | 2,00 | 2,00 |
| Glycerinmonostearat | 1,50 | 1,50 | 1,50 |
| PEG-30-Glycerylstearat | 2,00 | 2,00 | 2,00 |
| Glycerin | 3,00 | 3,00 | 3,00 |
| Isopropylpalmitat | 5,00 | 5,00 | 5,00 |
| Carbopol 980 (neutralisiert) | 0,30 | 0,30 | 0,30 |
| Sisterna SP 50 C | 1,00 | - | - |
| Sisterna L 70 C | - | 1,00 | - |
| Ryoto Sugar Ester S-270 | - | - | 1,00 |
| Parfum, Konservierungsstoffe | q.s. | q.s. | q.s. |
| Wasser, VES | ad 100,00 | ad 100,00 | ad 100,00 |

| Beispiel 5 | | | |
|---|---|---|---|
| Salbe | I | II | III |
| Vaseline | 36,00 | 36,00 | 36,00 |
| Ceresin | 10,00 | 10,00 | 10,00 |
| Zinkoxid | 4,00 | 4,00 | 4,00 |
| Pflanzliches Öl | 20,00 | 20,00 | 20,00 |
| Grilloten LSE 87 K | 0,02 | - | - |
| Ryoto Sugar Ester M 1695 | - | 0,02 | - |
| Sisterna SP 30 C | - | - | 0,02 |
| Parfum, Konservierungsstoffe | q.s. | q.s. | q.s. |
| Paraffinöl | ad 100,00 | ad 100,00 | ad 100,00 |

| Beispiel 6 | | | |
|---|---|---|---|
| Hautöl | I | II | III |
| Cetylpalmitat | 3,00 | 3,00 | 3,00 |
| C₁₂₋₁₅- Alkylbenzoat | 2,00 | 2,00 | 2,00 |
| Polyisobuten | 10,00 | 10,00 | 10,00 |
| Squalan | 2,00 | 2,00 | 2,00 |
| Grilloten LSE 87 K soft | 0,50 | - | - |
| Grilloten LSE 65 K | - | 0,50 | - |
| Cellobioselaurat | - | - | 0,50 |
| Parfum, Konservierungsstoffe | q.s. | q.s. | q.s. |
| Paraffinöl | ad 100,00 | ad 100,00 | ad 100,00 |

| Beispiel 7 | | | |
|---|---|---|---|
| Badeöl | I | II | III |
| Paraffinöl | 20,00 | 20,00 | 20,00 |
| PEG-40-hydriertes Rizinusöl | 5,00 | 5,00 | 5,00 |
| Lactobiosedistearat | 0,50 | - | - |
| Maltosemyristat | - | 0,50 | - |
| Ryoto Sugar Ester P 1570 | - | - | 0,50 |
| Parfum, Konservierungsstoffe | q.s. | q.s. | q.s. |
| Sojaöl | ad 100,00 | ad 100,00 | ad 100,00 |

| Beispiel 8 | | | |
|---|---|---|---|
| Lippenstift | I | II | III |
| Ceresin | 8,00 | 8,00 | 8,00 |
| Bienenwachs | 4,00 | 4,00 | 4,00 |
| Carnaubawachs | 2,00 | 2,00 | 2,00 |
| Vaseline | 40,00 | 40,00 | 40,00 |
| Hydriertes Rizinusöl | 4,00 | 4,00 | 4,00 |
| Caprylic/Capric Triglyceride | 6,00 | 6,00 | 6,00 |
| Cellobiosemyristat | 0,02 | - | 0,02 |
| Ryoto Sugar Ester M 595 | - | 0,02 | 0,02 |
| Ryoto Sugar Ester M 1695 | 0,02 | 0,02 | - |
| Ryoto Sugar Ester P 1570 | - | - | 0,50 |
| Parfum, Konservierungsstoffe | q.s. | q.s. | q.s. |
| Paraffinöl | ad 100,00 | ad 100,00 | ad 100,00 |

| Beispiel 9 | | | |
|---|---|---|---|
| Pflegemaske | I | II | III |
| PEG-50 Lanolin | 0,50 | 0,50 | 0,50 |
| Glycerylstearat | 2,00 | 2,00 | 2,00 |
| Sonnenblumenkernöl | 3,00 | 3,00 | 3,00 |
| Bentonit | 8,00 | 8,00 | 8,00 |
| Kaolin | 35,00 | 35,00 | 35,00 |
| Zinkoxid | 5,00 | 5,00 | 5,00 |
| Octaacetylmannobiose | 1,00 | - | - |
| Maltosemyristat | - | 1,00 | - |
| Saccharose-dioleat | - | - | 1,00 |
| Parfum, Konservierungsstoffe | q.s. | q.s. | q.s. |
| Wasser | ad 100,00 | ad 100,00 | ad 100,00 |

| Beispiel 10 | | | |
|---|---|---|---|
| Liposomenhaltiges Gel | I | II | III |
| Lecithin | 6,00 | 6,00 | 6,00 |
| Pflanzliches Öl | 12,50 | 12,50 | 12,50 |
| Hydrolysiertes Kollagen | 2,00 | 2,00 | 2,00 |
| Xanthan Gum | 1,40 | 1,40 | 1,40 |
| Butylenglycol | 3,00 | 3,00 | 3,00 |
| Sucrose-dilaurat | 0,30 | - | - |
| Sucrose-myristat | 0,30 | - | - |
| Sucrose-distearat | 0,30 | - | - |
| Sisterna SP 70 C | - | 0,30 | - |
| Sisterna SP 01 C | - | 0,30 | - |
| Ryoto Sugar Ester M 595 | - | 0,30 | - |
| Ryoto Sugar Ester S-270 | - | - | 0,30 |
| Grilloten PSE 141 G | - | - | 0,30 |
| Maltoselaurat | - | - | 0,30 |
| Parfum, Konservierungsstoffe | q.s. | q.s. | q.s. |
| Wasser | ad 100,00 | ad 100,00 | ad 100,00 |

| Beispiel 11 | | | |
|---|---|---|---|
| Duschpräparat mit Rückfetter | I | II | III |
| Cocoamidodiacetat | 10,00 | 10,00 | 10,00 |
| Natriumlaurylsulfat | 25,00 | 25,00 | 25,00 |
| Kalium Cocyl Hydrolysiertes Kollagen | 5,00 | 5,00 | 5,00 |
| Macadamianußöl | 5,00 | 5,00 | 5,00 |
| Natriumchlorid | 0,60 | 0,60 | 0,60 |
| Grilloten LSE 87 K | 0,30 | - | - |
| Sisterna L 70 C | - | 0,30 | - |
| Ryoto Sugar Ester M 1695 | - | - | 0,30 |
| Parfum, Konservierungsstoffe | q.s. | q.s. | q.s. |
| Wasser | ad 100,00 | ad 100,00 | ad 100,00 |

| Beispiel 12 | | | |
|---|---|---|---|
| Seifenstück | I | II | III |
| Na-Salz aus Talgfettsäuren | 60,00 | 60,00 | 60,00 |
| Na-Salz aus Kokosöl | 28,00 | 28,00 | 28,00 |
| Natriumchlorid | 0,50 | 0,50 | 0,50 |
| Grilloten LSE 65 K | 1,00 | - | - |
| Ryoto Sugar Ester S-270 | - | 1,00 | - |
| Sisterna L 70 C | - | - | 1,00 |
| Parfum, Konservierungsstoffe | q.s. | q.s. | q.s. |
| Wasser | ad 100,00 | ad 100,00 | ad 100,00 |

| Beispiel 13 | | | |
|---|---|---|---|
| Syndetseife | I | II | III |
| Natriumlaurylsulfat | 30,00 | 30,00 | 30,00 |
| Natriumsulfosuccinat | 10,00 | 10,00 | 10,00 |
| Kaliumcocoyl hydrolysiertes Kollagen | 2,00 | 2,00 | 2,00 |
| Dimethicon Copolyol | 2,00 | 2,00 | 2,00 |
| Paraffin | 2,00 | 2,00 | 2,00 |
| Maisstärke | 10,00 | 10,00 | 10,00 |
| Talcum | 10,00 | 10,00 | 10,00 |
| Glycerin | 3,00 | 3,00 | 3,00 |
| Grilloten LS 65 K | 1,00 | - | - |
| Sisterna SP 70 C | - | 1,00 | - |
| Sisterna SP 50 C | - | - | 1,00 |
| Parfum, Konservierungsstoffe | q.s. | q.s. | q.s. |
| Wasser | ad 100,00 | ad 100,00 | ad 100,00 |

| Beispiel 14 | | | |
|---|---|---|---|
| Haarpflegemittel | I | II | III |
| TEA-Cocoyl hydrolysiertes Kollagen | 30,00 | 30,00 | 30,00 |
| Monoethanolaminlaurylsulfat | 25,00 | 25,00 | 25,00 |
| Mandelöl | 2,00 | 2,00 | 2,00 |
| Natriumchlorid | 1,00 | 1,00 | 1,00 |
| Grilloten LSE 65 K soft | 1,00 | - | - |
| Sucroselaurat | - | 1,00 | - |
| Ryoto Sugar Ester M 1695 | - | - | 1,00 |
| Parfum, Konservierungsstoffe | q.s. | q.s. | q.s. |
| Wasser | ad 100,00 | ad 100,00 | ad 100,00 |

| Beispiel 15 | | | |
|---|---|---|---|
| Pflegeshampoo | I | II | III |
| Natriumlaurylsulfat | 34,00 | 34,00 | 34,00 |
| Dinatriumlaurylsulfosuccinat | 6,00 | 6,00 | 6,00 |
| Cocoamidopropylbetain | 10,00 | 10,00 | 10,00 |
| Glycoldistearat | 5,00 | 5,00 | 5,00 |
| Sucroselaurat | 0,02 | - | - |
| Mannooselaurat | - | 1,00 | - |
| Cellobiosemyristat | - | - | 1,00 |
| Parfum, Konservierungsstoffe | q.s. | q.s. | q.s. |
| Wasser | ad 100,00 | ad 100,00 | ad 100,00 |

| Beispiel 16 | | | |
|---|---|---|---|
| Haarkur | I | II | III |
| Cetylalkohol | 5,00 | 5,00 | 5,00 |
| Caprylic/Capric Triglyceride | 3,00 | 3,00 | 3,00 |
| Petrolatum | 2,00 | 2,00 | 2,00 |
| Wollwachsalkohol | 0,50 | 0,50 | 0,50 |
| Sisterna SP 50 C | 1,00 | - | - |
| Grilloten LSE 65 K | - | 1,00 | - |
| Ryoto Sugar Ester L 1695 | - | - | 1,00 |
| Parfum, Konservierungsstoffe | q.s. | q.s. | q.s. |
| Wasser | ad 100,00 | ad 100,00 | ad 100,00 |

| Beispiel 17 | | | |
|---|---|---|---|
| Haarspülung | I | II | III |
| Cocoamidopropylbetain | 5,00 | 5,00 | 5,00 |
| Cetylalkohol | 2,00 | 2,00 | 2,00 |
| Propylenglycol | 2,00 | 2,00 | 2,00 |
| Citronensäure | 0,30 | 0,30 | 0,30 |
| Grilloten PSE 141 G | 1,00 | - | - |
| Sisterna SP 01 C | - | 1,00 | - |
| Ryoto Sugar Ester S-370 | - | - | 1,00 |
| Parfum, Konservierungsstoffe | q.s. | q.s. | q.s. |
| Wasser | ad 100,00 | ad 100,00 | ad 100,00 |

| Beispiel 18 | | | |
|---|---|---|---|
| Haarfestiger | I | II | III |
| Polyvinylpyrrolidon/Vinylacetat/ | 5,00 | 5,00 | 5,00 |
| Vinylpropionat-Copolymer | | | |
| Ethanol | 45,00 | 45,00 | 45,00 |
| Grilloten LSE 87 K | 1,00 | - | - |
| Sisterna SP 30 C | - | 1,00 | - |
| Ryoto Sugar Ester M 595 | - | - | 1,00 |
| Parfum, Konservierungsstoffe | q.s. | q.s. | q.s. |
| Wasser | ad 100,00 | ad 100,00 | ad 100,00 |

| Beispiel 19 | | | |
|---|---|---|---|
| Frisiercrème | I | II | III |
| Vaseline | 4,00 | 4,00 | 4,00 |
| Cetearylalkohol | 4,00 | 4,00 | 4,00 |
| PEG-40-hydriertes Rizinusöl | 2,00 | 2,00 | 2,00 |
| Isopropylpalmitat | 5,00 | 5,00 | 5,00 |
| Citronensäure | 1,00 | 1,00 | 1,00 |
| Grilloten PSE 141 G | 0,30 | - | - |
| Sisterna L 70 C | 0,30 | - | - |
| Sisterna SP 50 C | - | 0,30 | - |
| Ryoto Sugar Ester L 1695 | - | 0,30 | - |
| Maltoselaurat | - | - | 0,30 |
| Ryoto Sugar Ester M 1695 | - | - | 0,30 |
| Parfum, Konservierungsstoffe | q.s. | q.s. | q.s. |
| Wasser | ad 100,00 | ad 100,00 | ad 100,00 |

| Beispiel 20 | | | |
|---|---|---|---|
| Rasierschaum | I | II | III |
| Stearinsäure | 7,00 | 7,00 | 7,00 |
| Natriumlaurylsulfat | 3,00 | 3,00 | 3,00 |
| Stearylalkohol | 1,00 | 1,00 | 1,00 |
| Glycerin | 5,00 | 5,00 | 5,00 |
| Triethanolamin | 3,60 | 3,60 | 3,60 |
| Grilloten PSE 141 G | 0,30 | - | - |
| Octaacetylmannobiose | - | 0,30 | - |
| Sucroselaurat | - | - | 0,30 |
| Parfum, Konservierungsstoffe | q.s. | q.s. | q.s. |
| Wasser | ad 100,00 | ad 100,00 | ad 100,00 |

| Beispiel 21 | | | |
|---|---|---|---|
| Fußcrème | I | II | III |
| Soluan 5 | 2,00 | 2,00 | 2,00 |
| Methylsalicylat | 5,00 | 5,00 | 5,00 |
| Caprylic/Capric Triglyceride | 10,00 | 10,00 | 10,00 |
| Stearinsäure | 5,00 | 5,00 | 5,00 |
| Cetylalkohol | 1,00 | 1,00 | 1,00 |
| Glycerin | 2,00 | 2,00 | 2,00 |
| Dimethicon | 1,00 | 1,00 | 1,00 |
| Carbopol 984 | 0,50 | 0,50 | 0,50 |
| Triethanolamin | 1,50 | 1,50 | 1,50 |
| Sucroselaurat | 1,00 | - | - |
| Sucrosecaprylat | - | 1,00 | - |
| Sucrosecaprat | - | - | 1,00 |
| Parfum, Konservierungsstoffe | q.s. | q.s. | q.s. |
| Wasser | ad 100,00 | ad 100,00 | ad 100,00 |

| Beispiel 22 | | | |
|---|---|---|---|
| Aerosolspray | I | II | III |
| Octyldodecanol | 0,50 | 0,50 | 0,50 |
| Sisterna L 70 C | 0,20 | - | - |
| Ryoto Sugar Ester M 595 | - | 0,20 | - |
| Grilloten LSE 65 K | - | - | 0,20 |
| Parfum, Konservierungsstoffe | q.s. | q.s. | q.s. |
| Ethanol | ad 100,00 | ad 100,00 | ad 100,00 |

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

| Beispiel 23 | | | |
|---|---|---|---|
| Pumpspray | I | II | III |
| PEG-40-Hydriertes Rizinusöl | 2,00 | 2,00 | 2,00 |
| Glycerin | 1,00 | 1,00 | 1,00 |
| Grilloten LSE 65 K soft | 0,20 | - | - |
| Sisterna SP 01 C | - | 0,20 | - |
| Ryoto Sugar S-270 | - | - | 0,20 |
| Parfum, Konservierungsstoffe | q.s. | q.s. | q.s. |
| Wasser | ad 100,00 | ad 100,00 | ad 100,00 |

| Beispiel 24 | | | |
|---|---|---|---|
| Roll-on-Gel | I | II | III |
| 1,3-Butylenglycol | 2,00 | 2,00 | 2,00 |
| PEG-40-Hydriertes Rizinusöl | 2,00 | 2,00 | 2,00 |
| Hydroxyethylcellulose | 0,50 | 0,50 | 0,50 |
| Sisterna SP 70 C | 0,30 | - | - |
| Ryoto Sugar Ester M 595 | 0,30 | - | - |
| Grilloten LSE 87 K | - | 0,30 | - |
| Sisterna SP 30 C | - | 0,30 | - |
| Ryoto Sugar Ester L 1695 | - | - | 0,30 |
| Grilloten LSE 65 K | - | - | 0,30 |
| Parfum, Konservierungsstoffe | q.s. | q.s. | q.s. |
| Wasser | ad 100,00 | ad 100,00 | ad 100,00 |

| Beispiel 25 | | | |
|---|---|---|---|
| Roll-on-Emulsion | I | II | III |
| Tricetearethphosphat | 0,30 | 0,30 | 0,30 |
| Octyldodecanol | 2,00 | 2,00 | 2,00 |
| C₁₂₋₁₅-Alkylbenzoat | 2,00 | 2,00 | 2,00 |
| C₁₀₋₃₀-Alkylacrylat | 0,15 | 0,15 | 0,15 |
| Ryoto Sugar Ester M 595 | 0,30 | - | - |
| Ryoto Sugar Ester S-270 | - | 0,30 | - |
| Sisterna L 70 C | - | - | 0,30 |
| Parfum, Konservierungsstoffe | q.s. | q.s. | q.s. |
| Wasser | ad 100,00 | ad 100,00 | ad 100,00 |

| Beispiel 26 | | | |
|---|---|---|---|
| Wachsstift | I | II | III |
| Hydriertes Rizinusöl | 5,00 | 5,00 | 5,00 |
| Bienenwachs | 6,00 | 6,00 | 6,00 |
| Ceresin | 30,00 | 30,00 | 30,00 |
| C₁₂₋₁₅-Alkylbenzoat | 17,00 | 17,00 | 17,00 |
| Sisterna SP 70 C | 0,50 | - | - |
| Ryoto Sugar ester L 1695 | - | 0,50 | - |
| Grilloten LSE 87 K soft | - | - | 0,50 |
| Parfum, Konservierungsstoffe | q.s. | q.s. | q.s. |
| Octyldodecanol | ad 100,00 | ad 100,00 | ad 100,00 |

## Patentansprüche

1. Verwendung einer Verbindung oder mehrerer Verbindungen aus der Gruppe der mit einem oder mehreren Fettsäureresten veresterten Di- und Oligosaccharide als antiadhäsive Wirkstoffe gegenüber Mikroorganismen, Viren, Parasiten und Protozoen.

2. Verwendung gemäß Anspruch 1, dadurch gekennzeichnet, daß die Fettsäurekomponenten der veresterten Di- und Oligosaccharide gesättigte und/oder ein bis fünffach ungesättigte Mond Di-, Tri-, Tetra-, Penta- und Hexacarbonsäuren darstellen.

3. Verwendung gemäß Anspruch 1, dadurch gekennzeichnet, daß die veresterten Di- und Oligosaccharide gekennzeichnet sind durch die Struktur 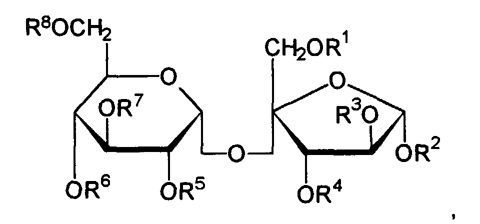 wobei R¹ - R⁸ vorteilhaft unabhängig voneinander gewählt werden können aus der Gruppe aus Wasserstoffatomen und verzweigten und unverzweigten Alkanoylgruppen mit 1 - 24 Kohlenstoffatomen

4. Verwendung gemäß Anspruch 3, dadurch gekennzeichnet, daß bis zu sieben der Reste R¹ - R⁸ Wasserstoffatome und einer bis zu acht der der Reste R¹ - R⁸ gewählt werden aus der Gruppe der verzweigten und unverzweigten Alkanoylgruppen mit 8 - 20 Kohlenstoffatomen, davon insbesondere der Lauroylrest und der Myristoylrest.
